# EUROPEAN PATENT SPECIFICATION

(11) **EP 2 448 828 B1**
(45) Date of publication and mention of the grant of the patent: **11.12.2013**
(21) Application number: 10794442.3
(22) Date of filing: 24.06.2010
(51) Int. Cl.: B65B 31/02, B65B 55/02, B65B 55/10, A61L 2/20

(54) **PACKAGING MACHINE AND PACKAGING METHOD**
VERPACKUNGSMASCHINE UND VERPACKUNGSVERFAHREN
MACHINE D'EMBALLAGE ET PROCEDE D'EMBALLAGE

(30) Priority: 03.07.2009 SE 0900909
(43) Date of publication of application: 09.05.2012
(73) Proprietor: Tetra Laval Holdings & Finance S.A., 1009 Pully (CH)
(72) Inventor: LINDBLAD, Ulf, 224 56 Lund (SE); OLSSON, Jenny, 237 33 Bjärred (SE)
(74) Representative: Tetra Pak - Patent Attorneys SE
(86) International application number: PCT/SE2010/000181
(87) International publication number: WO 2011/002384

(56) References cited:
- WO-A1-2004/054883
- WO-A1-2010/044025
- DE-A1-102004 012 113
- NL-A- 8 701 293
- SE-C2- 511 117
- US-A- 3 486 295
- US-A- 4 597 192
- US-A- 4 693 052
- US-A- 5 865 010
- US-A1- 2006 185 321

## Description

### Technical Field

The present invention relates to a packaging machine and a packaging method. The packaging machine comprises a filling zone for filling packages through a respective open end thereof, a sealing zone for sealing said respective open end of the packages after filling, a final folding zone for forming the packages after sealing and a conveyor for transporting the packages through said zones in a transport direction, (see e.g. US 5 865 010).

### Technical Background

Within the food industry, beverages and other products are often packed in paper or paperboard based packages. Packages intended for liquid food are often produced from a packaging laminate comprising a core layer of paper or paperboard and an outer, liquid-tight layer of thermoplastic material on at least that side of the core layer which will form the inside of the packages.

One kind of frequently occurring packages are the so-called carton bottles. In substance, these are composed of a lower part in the form of a sleeve of packaging laminate like the one described above, and an upper part in the form of a plastic top having a neck which is provided with an opening/closing means, such as a screw cap.

In a known packaging machine, carton bottles which are open in the bottom, i.e. the sleeve end, are produced. Then, the open carton bottles are transported, disposed upside-down, to a sterilization station for at least inside sterilization in order to extend the shelf-life of the product to be packed in the carton bottles. Depending upon the desired length of shelf-life, and depending upon whether the carton bottles are to be distributed and stored in a refrigerated environment or at room temperature, different levels of sterilization may be selected. After sterilization the carton bottles are further transported to a filling station for product filling, a sealing station for sealing of the open bottom and a final folding station for final folding of the bottom. For the transportation through the packaging machine, the packages are arranged in carriers disposed on an endless conveyor belt running through the different stations.

In order not to risk recontamination of the inside of the carton bottles, it is important to maintain an aseptic environment in the packaging machine in a section after the sterilization station until bottom sealing has been effected. For various reasons that will not be discussed in detail here, this is not very easily achieved.

In other parts of the packaging machine, smaller demands are made on the conditions prevailing. For example in the final folding station, not aseptic but yet hygienic conditions should be maintained to enable a safe operation of the packaging machine. If such hygienic conditions are not maintained here, there is a risk of contamination of the conveyor and the carriers which is undesired since the conveyor and the carriers, after outfeed of finished carton bottles, should be reloaded with new open carton bottles for performance of the steps above.

However, it is not very easy to maintain the hygienic conditions inside the final folding station. This is, inter alia, a result of all the free particles, e.g. paper dust, that are formed in connection with the final folding operation. Further, these particles may be spread to other parts of the packaging machine and jeopardize the desired conditions also there. As a result thereof, relatively frequent stops for performance of machine cleaning and sterilization are required. Additionally, the final folding station is relatively error-prone compared to the rest of the stations of the packaging machine, inter alia due to the mechanical complexity of the final folding means. When an error, such as a jam, occurs inside the final folding station, an external intervention is required for error remedy. In connection therewith, the packaging machine must be stopped. Further, an external intervention often results in a disturbance of the conditions inside the final folding station and probably also in other parts of the packaging machine. Therefore, any such operation must be followed by a time-consuming machine cleaning and sterilization operation. In all, this results in considerable machine downtime.

### Summary

An object of the present invention is to provide a packaging machine and method which, at least partly, eliminate potential limitations of prior art. The basic concept of the invention is to protect the desired conditions within different parts of the packaging machine by marking off an area for final folding of the packages, which area thereby is separated from other parts of the packaging machine, also from a conveyor for package transport through the machine. By such a markingoff, a lower hygiene level can be allowed in the area for package final folding. Consequently, the number of necessary machine cleaning and sterilization operations will decrease which, in turn, will increase the machine uptime.

The packaging machine and method for achieving the object above are defined in the appended claims and discussed below.

A packaging machine according to the present invention comprises a filling zone for filling packages through a respective open end thereof, a sealing zone for sealing said respective open end of the packages after filling, a final folding zone for forming the packages after sealing and a conveyor for transporting the packages through said zones in a transport direction. The packaging machine is characterized in that the conveyor is arranged to run outside the final folding zone and instead run through a buffer zone to transport the packages through the final folding zone. Further, it is characterized in that it comprises means for maintaining a first pressure inside the sealing zone, a second pressure inside the final folding zone and a third pressure inside the buffer zone. The first and third pressures are higher than the second pressure which, in turn, is higher than a fourth pressure prevailing outside the packaging machine.

Of course, the packaging machine can be used in connection with different types of packages, such as carton bottles of the above described type.

Since the conveyor is excluded from the final folding zone, it is protected from the environment prevailing therein, which environment should be cleaner than the surrounding but which may have a lower hygienic level than the rest of the machine, e.g. due to paper dust and other free particles present therein and formed in connection with the final folding operation. The lower hygienic level may also result from an external intervention, by manual operations, in the final folding zone necessary to take care of an error that has occurred therein.

Even if the conveyor is arranged to run outside the final folding zone it can still convey the packages through the same in that the packages protrudes from the conveyor and into the final folding zone from the buffer zone, a part of the packages being received in the buffer zone whereas the remaining part of the packages being received in the final folding zone.

The final folding, buffer and sealing zones are separated or delimited from each other by maintaining different pressures in the different zones. Since the pressure inside the final folding zone is lower than the pressure inside the sealing and buffer zones, airborne particles from the final folding zone are prevented from reaching the sealing and buffer zones. This means that the number of frequent, scheduled stops for performance of machine cleaning and sterilization can be reduced. It also means that a short-stop of the packaging machine, not demanding any subsequent cleaning and sterilization operations, may be enough in connection with an external intervention inside the packaging machine, e.g. for an error remedy. In all, this increases the machine capacity. Further, since the pressure outside the packaging machine is lower than the pressure inside the final folder, "unclean" surrounding air is prevented from leaking into the final folding zone from outside of the packaging machine. Naturally, this aids in maintaining the hygienic conditions of the packaging machine.

According to one embodiment of the present invention, the packaging machine further comprises an outfeed station for output of the sealed, filled and formed packages, the means for pressure maintenance being further arranged to maintain a fifth pressure inside the outfeed station which is higher than the fourth pressure prevailing outside the packaging machine and lower than the second and third pressures inside the final folding zone and buffer zone, respectively. The outfeed station accommodates means for collecting the formed packages from the final folding zone and delivering them outside the packaging machine. Just like the final folding means, these collecting and delivering means are relatively mechanically complex, and therefore relatively error prone. Thus, external intervention is relatively often required for error remedy with a following risk of recontamination of the environment inside the outfeed station. Further, not even under normal, well-functioning circumstances is it very easy to maintain the hygienic conditions inside the outfeed station, inter alia, because of its communication with the surroundings. However, by securing the above specified relation between the fifth pressure inside the outfeed station and the second, third and fourth pressures, airborne particles inside the outfeed station can be prevented from reaching the final folding zone and the buffer zone and disturb the desired conditions therein. Instead, they will be forced out of the packaging machine.

The inventive packaging machine may be so constructed that the final folding zone occupies an upper part thereof and the buffer zone occupies a lower part thereof, said lower part being arranged below said upper part. This embodiment means that the conveyor is arranged to run below the final folding zone and that the packages are arrange to project into the final folding zone from below.

According to one embodiment of the present invention, the buffer zone also occupies a portion of the packaging machine arranged between the sealing zone and said upper and lower parts of the packaging machine, the buffer zone therby delimiting the final folding zone from the sealing zone. This construction further promotes the maintainance of the desired conditions within the different zones of the packaging machine.

In accordance with one embodiment of the present invention, the means for maintaining the different pressures inside the different zones includes a supply device for supplying a flow of cleaned air to the packaging machine outside the final folding zone, and a separation device for physically delimiting the final folding zone from the rest of the zones. Further, the separation device has an opening for enabling passage of the packages through the final folding zone.

The air provided to the packaging machine can have different degrees of purity depending on the specific application. As an example, the air to be provided to the packaging machine could be sterile in order to be able to achieve aseptic and hygienic conditions in parts of the packaging machine.

The opening of the separation device could be formed in a number of different ways. As an example, the opening could be formed as a slit in the separation device for receiving the packages protruding from the conveyor.

In accordance with one embodiment of the present invention, the separation device comprises a first baffle and a second baffle delimiting the final folding zone from the buffer zone. Use of baffles for this purpose enables a mechanically simple inventive construction which is relatively easy to clean.

The above baffles can be arranged in a number of different ways. As an example, the first baffle could extend essentially orthogonally to the transport direction whereas the second baffle could extend essentially parallel to the transport direction. Further, the first baffle could extend from a top wall towards a bottom wall of the packaging machine, whereas the second baffle could extend from the first baffle in the transport direction. Further, the first and second baffles could extend essentially all the way from one side wall to another side wall of the packaging machine. The features above, and combinations thereof, provide a particularly mechanically simple construction of the packaging machine according to the present invention.

In accordance with one embodiment of the present invention, the packaging machine further comprises a hatch in an area of the final folding zone for external access thereto. As was mentioned by way of introduction, if the packaging machine is malfunctioning, this is not seldom due to an error in the final folding zone. This embodiment is advantageous since it provides easy access to the final folding zone and enables a fast and smooth remedy of such an error. In turn, this increases the machine capacity. Further, as mentioned above, since the design of the packaging machine is such that the pressure inside the final folding zone is higher than the outside pressure and lower than the sealing zone pressure and the buffer zone pressure, opening of this hatch does not result in destruction of the hygienic and possibly aseptic conditions in different parts of the packaging machine. Instead, when the hatch is opened, airborne particles from inside the final folding zone can be blown out of the packaging machine through the hatch. Further, the over pressure inside the final folding zone constitutes an obstacle to the outside air to enter the final folding zone and thereby the packaging machine. Thereby, an external access to the final folding zone of the inventive packaging machine need not be followed by a time and work consuming machine cleaning and sterilization operation which is a huge advantage from an economical point of view.

A packaging method according to the present invention comprises filling, in a filling zone, packages through a respective open end thereof, sealing, in a sealing zone, said respective open end of the packages after filling, forming, in a final folding zone, the packages after sealing and transporting the packages on a conveyor through said zones in a transport direction. The zones are comprised in a packaging machine. The packaging method is characterized in that it further comprises running the conveyor outside the final folding zone and instead running it through a buffer zone to transport the packages through the final folding zone. Further, it is characterized in that it comprises maintaining a first pressure inside the sealing zone, a second pressure inside the final folding zone and a third pressure inside the buffer zone. The first and third pressures are higher than the second pressure which, in turn, is higher than a fourth pressure prevailing outside the packaging machine.

The characteristics discussed in connection with the inventive packaging machine are, of course, transferable to the inventive packaging method. Further, these characteristics may naturally be combined in the same embodiment.

### Brief Description of the Drawings

Fig. 1 is a schematic side view of part of a packaging machine according to the present invention.
Fig. 2 schematically illustrates a cross section of the packaging machine of Fig. 1, taken along the line A-A.
Fig. 3 schematically illustrates a cross section of a part of the packaging machine of Fig. 1, taken along the line B-B.
Fig. 4 is a schematic side view of a section of the packaging machine part shown in figure 1.
Fig. 5 corresponds to figure 2 and schematically illustrates an alternative embodiment of the present invention.
Fig. 6 corresponds to figure 3 and schematically illustrates said alternative embodiment together with fig. 5.

### Detailed Description

In the following, the term (adequate or the like) sterile is taken to signify that the package, after sterilization, attains a level of sterilization which is design-nated commercially sterile.

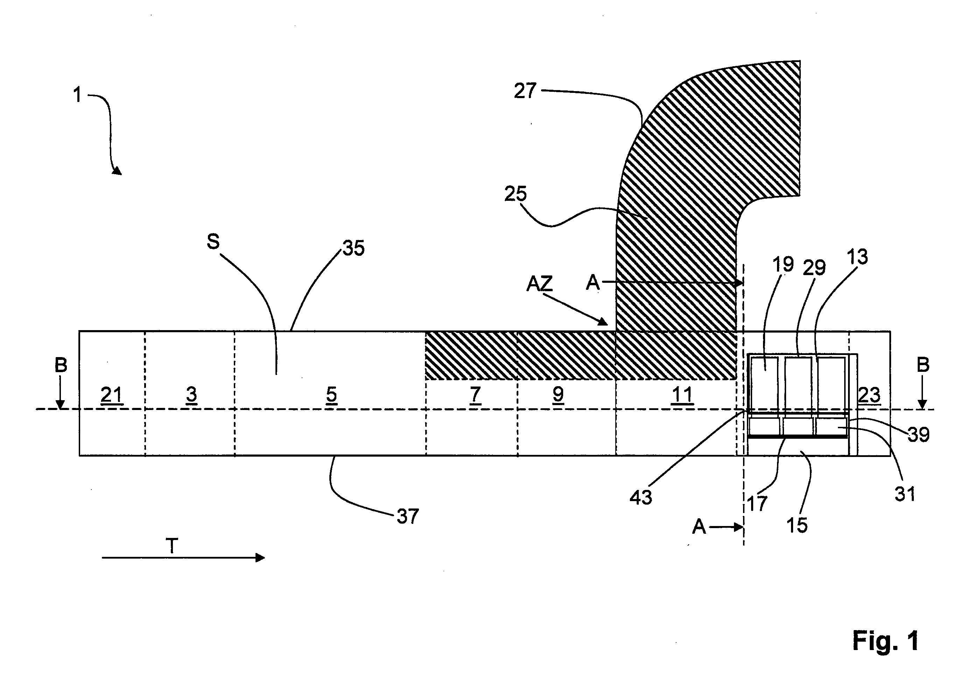

Figure 1 illustrates a packaging machine 1 (not illustrated in its entirety) according to one embodiment of the present invention for producing filled, finished packages in the form of carton bottles of the initially described type. The packaging machine 1 is adapted for gas phase sterilization of the carton bottles prior to filling, sealing and folding of the same. To this end, the packaging machine 1 comprises a preheating zone 3, a gassing zone 5, a venting zone 7, a filling zone 9, a sealing zone 11, a final folding zone 13 and a buffer zone 15. Additionally, the packaging machine 1 comprises an endless conveyor 17 for transporting the carton bottles 19 through the various zones in a transport direction T, the conveyor running back through the packaging machine in a return direction which is essentially opposite to the transport direction T (not illustrated in figure 1). The zones 3-9 are arranged in a row at the end of which the sealing zone 11, the final folding zone 13 and the buffer zone 15 are arranged, as is illustrated in the figure. The buffer zone 15 extends below the final folding zone 13, between the final folding zone and the sealing zone 11 and behind the sealing zone 11 seen in the direction of figure 1. The specific extension of the buffer zone 15 is illustrated in Swedish copending patent application filed by the applicant on the same date as the present application and titled "Packaging machine and packaging method II" (SE-0900910-1). The conveyor runs through all zones but the final folding zone 13, which will be further discussed hereinafter. Further, the packaging machine 1 is "open" by nature and has an infeed station 21 and an outfeed station 23 for the carton bottles 19 which are open towards the machine surround-dings. The outfeed station 23 accommodates means (not illustrated) for collecting the folded packages from the final folding zone 13 and delivering them outside the packaging machine. The infeed and outfeed stations will not be described in detail herein. The same goes for an arrangement 25, comprising inter alia a duct 27 extending into the packaging machine to enclose the sealing zone 11, for supplying sterile air to the rest of the packaging machine 1, which arrangement is described in detail in Swedish copending patent application filed by the applicant on the same date as the present application and titled "Device for cleaned air provision" (SE-0900908-5), which application is hereby incorporated herein by reference. The boundaries between the zones, and the zones and the stations, have been illustrated with broken lines in the figures. For illustrative purposes, as apparent from the figure, the packaging machine 1 has been opened up (a side wall S has been partly removed) in the area the final folding zone 13, the buffer zone 15 and the outfeed station 23.

As indicated by the name, the carton bottles 19 are fed, with their bottom ends 29 open, to the packaging machine 1 via the infeed station 21 where they are arranged upside-down, with their respective bottom end 29 directed upwards, in carrier means 31 attached to the conveyor 17. Arranged like this, the carton bottles are then moved through the zones 3, 5 and 7 for sterilization. In the gassing zone 5, the carton bottles 19 are exposed to gaseous hydrogen peroxide. In order to prevent the hydrogen peroxide from condensing on the surface of the carton bottles in the gassing zone 5, which impedes later removal, the carton bottles are heated up in the preheating zone 3 to a temperature above the dew point of the hydrogen peroxide gas. In the venting zone 7, the carton bottles are subjected to sterile hot air in order to vent off hydrogen peroxide which remains in and on the carton bottles. This sterilization operation is described in more detail in Swedish copending patent application filed by the applicant on the same date as the present application and titled "A device and a method for sterilization of packages" (SE-0900907-7), which application is hereby incorporated herein by reference. After sterilization, the carton bottles are fed to the filling zone 9 for filling of the desired product, the sealing zone 11 for bottom sealing and the final folding zone 13, and thereby the buffer zone 15, for final forming. Finally, the finished, filled carton bottles are output from the packaging machine 1 via the outfeed station 23.

In order not to risk recontamination of the carton bottles, or at least the inside thereof, after sterilization agent exposure, it is important to maintain an aseptic environment at least in an upper part of the packaging machine 1 from the venting zone 7 to the sealing zone 11, i.e. until the carton bottles 19 have been finally sealed. This upper part extends somewhere from above the conveyor 17 to the top of the packaging machine 1 and is arranged to receive the bottom ends 29 of the carton bottles 19 when these are conveyed through the apparatus 1. This upper part is called an aseptic zone, AZ, and is illustrated in figure 1 (scored area).

The endless conveyor 17 with the carriers 31 is, after delivery of finished carton bottles at the outfeed station 23, arranged to travel back through the packaging machine to the infeed station 21 to be loaded with new unfinished carton bottles for treatment in the above described way, without being cleaned in between, except for in connection with scheduled, regular productions stops intended for machine cleaning and sterilization. In view of this, it is important to also keep a check on the conditions, in respect of hygiene, maintained throughout the rest of the packaging machine, outside the aseptic zone. The final folding zone 13 is one of the parts of the packaging machine where it is most difficult to maintain hygienic conditions. This is because of the final folding operation generating paper dust and other free particles that can disturb the hygiene inside the final folding zone, and also inside other parts of the machine, if preventive measures are not taken. The outfeed station 23 is another part of the packaging machine where it is relatively difficult to maintain hygienic conditions. This is because of the outfeed station being open towards the surroundings to allow package output, which surroundings not always are very clean. Thus, particles from the outside can disturb the desired conditions inside the outfeed station and the rest of the packaging machine if preventive measures are not taken. Further, if the packaging machine is not working properly, this is often due to a problem, such as a jam, in the final folding zone or the outfeed station, i.e. a malfunctioning of the means for performing the final folding operation or the package outfeed, which means will not be described herein. To take care of such a problem, an external fixing operation is often required. Such an external intervention can also disturb the hygiene inside the final folding zone, the outfeed station and other packaging machine parts if proper measures are not taken.

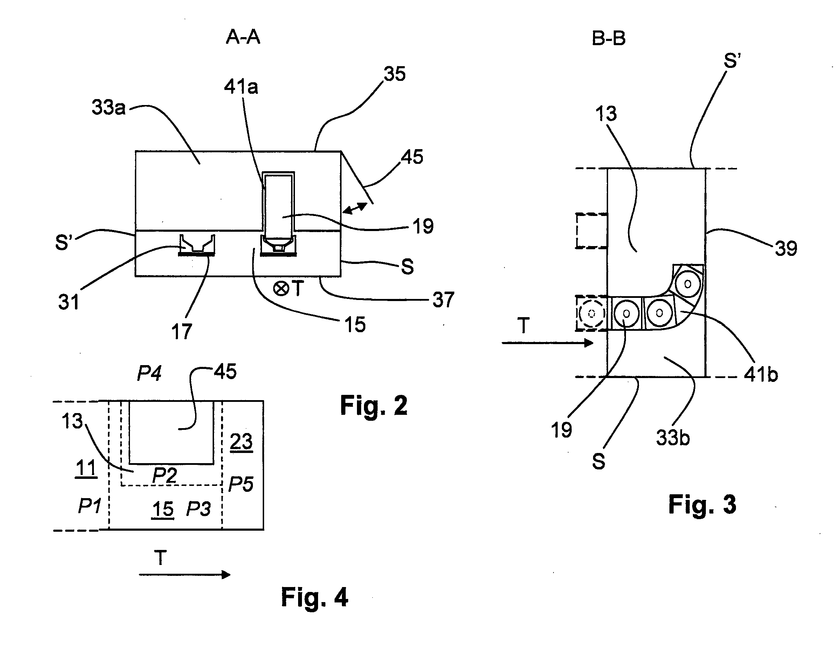

Figure 2 schematically shows a cross section of the packaging machine 1 taken along the line A-A in figure 1. Figure 3 schematically shows a part of a cross section of the packaging machine 1 taken along the line B-B in figure 1, which part corresponds to the area of the final folding zone 13 and a part of the buffer zone 15. These two figures, which are simplified to a high extent (irrelevant elements have, inter alia, been omitted) illustrate the characteristic features of the present invention in more detail. As apparent from the figures, the packaging machine 1 comprises a separation device 33 (33a + 33b) for physically separating the final folding zone 13 from the rest of the zones. The separation device includes a first baffle 33a extending through the packaging machine 1 orthogonal to the transport direction T to delimit the final folding zone 13 from the buffer zone 15 in an essentially vertical direction. The first baffle 33a extends from a top wall 35 and a predetermined distance towards a bottom wall 37 and all the way from one side wall S to the other side wall S' of the packaging machine 1. Further, the separation device includes a second baffle 33b extending through the packaging machine 1 parallel to the transport direction T to delimit the final folding zone 13 from the buffer zone 15 in an essentially horizontal direction. In fact, for draining purposes, the second baffle 33b is arranged very slightly angled in relation to the horizontal direction, which will not be further discussed herein. The second baffle 33b is connected to the first baffle 33a and extends therefrom all the way to a plate 39 separating the buffer zone 15 from the outfeed station 23 and all the way from one side wall S to the other side wall S' of the packaging machine 1. The plate 39 extends all the way from the top wall 35 to the bottom wall 37 and from the side wall S to the other side wall S' of the packaging machine. However, to allow output of the finally formed packages, the plate has an opening (not illustrated) to the outfeed station 23. The separation device 33 has an opening 41 (41a+41b) to allow passage of the carton bottles 19 through the final folding zone 13. The opening is preferably as small as possible and adapted to the particular shape of the packages and the path of the conveyor. Naturally, the opening 41 extends through both the first and the second baffle, 33a and 33b, respectively. In the first baffle 33a, it has the form of a rectangular slot 41 a extending from a connection line 43 between the baffles a predetermined distance towards the top wall 35 of the packaging machine 1. In the second baffle 33b, it has the form of a J-shaped slot 41b extending from the connection line 43 to the plate 39. Since the carriers 31 are empty after package output at the outfeed station 23, no slot is necessary for the return direction of the conveyor. The sealing zone 11 is separated from the buffer zone 15 by a wall of the duct 27 of the arrangement 25.

As mentioned above, the conveyor 17 is arranged to run outside the final folding zone 13 and instead through the buffer zone 15 to transport the carton bottles 19 through the final folding zone. In accordance therewith, the conveyor runs below the second baffle 33b and follows the slot 41 b formed therein so that the carton bottles, when arranged in the carriers 31 on the conveyor, protrudes through the slot and into the final folding zone 13. How big part of the carton bottles that protrudes into the final folding zone is naturally dependent upon the distance between the conveyor and the second baffle.

The packaging machine comprises a hatch 45 (shown only in figures 2 and 4) in the area of the final folding zone 13 for easy external access to the same. This hatch consists of a section of the side wall S, which section is hinged at the top wall 35 of the packaging machine 1. The hatch 45 is illustrated in figure 2 in its opened position and in figure 4 in its closed position. Figure 4 is similar to figure 1 but shows only the part of the packaging machine 1 corresponding to the final folding zone 13, a part of the buffer zone 15 and the outfeed station 23 in a non-opened-up condition. During normal operation of the packaging machine 1, the hatch 45 is closed. However, if a functional error arises in the packaging machine 1, more particularly in the final folding zone 13 thereof, the hatch 45 can easily be opened for quick access to the final folding zone, and thereby the final folding means, for smooth remedy of the error.

The above mentioned arrangement 25 for sterile air supply together with the separation device 33, the plate 39 and the wall of the duct 27 generate pressure differences inside the packaging machine 1. More particularly, a first pressure P1 is maintained inside the sealing zone 11 and a third pressure P3 maintained inside the buffer zone 15 is higher than a second pressure P2 maintained inside the final folding zone 13 which is higher than a fifth pressure P5 maintained inside the outfeed station 23, which in turn is higher than a fourth pressure P4 prevailing outside the packaging machine 1. Further, the pressure inside the sealing zone is higher than the pressure inside the buffer zone which means that the following condition is fulfilled: P1>P3>P2>P5>P4. This relationship results in the following spontaneous flow through the packaging machine when the hatch is closed: sealing zone → buffer zone → final folding zone → outfeed station → machine surroundings. When the hatch is open, the following flow will instead prevail: sealing zone → buffer zone → final folding zone → machine surroundings (possibly via the outfeed station).

Thus, as explained above, it is important to keep a check on the hygiene conditions in the packaging machine outside the aseptic zone. By screening off the final folding zone 13 (and also the outfeed station) from the rest of the packaging machine, in the earlier described way, it is possible to obtain the above stated pressure condition. In turn, this pressure condition result in that any free particles, such as dust, formed inside the final folding zone, is prevented from entering the buffer zone 15 and the sealing zone 11, and thereby the rest of the zones. Since the conveyor is not running through the final folding zone but through the buffer zone, also this and the carriers will be protected. Further, if it is necessary to access the final folding zone from the outside, for example to fix an error therein, this can easily be done by opening the access hatch 45. Because of the higher pressure inside the final folding zone compared to the outside pressure, such a hatch opening will result in that air is automatically flowing from inside of the final folding zone to the outside rather than the other way round. Dirty air is thereby prevented from entering the packaging machine. Nevertheless, if particles from the outside should be introduced into the final folding zone in connection with fixing the error, these particles would be prevented from reaching the buffer and sealing zones because of the prevailing pressure condition. Thus, no machine cleaning or sterilization operations will automatically be necessary after an external intervention into the packaging machine according to the present invention. Thus, a short-stop of the packaging machine may suffice for error remedy.

The above described embodiment should only be seen as an example. A person skilled in the art realizes that this embodiment can be modified and varied in a number of ways without deviating from the inventive conception.

As an example, in the above described embodiment, the hatch is formed as a part of the side wall of the packaging machine. The hatch could of course be constructed in alternative ways. It could, e.g. be formed as a part of the top wall of the packaging machine.

Further, the separation device comprised in the packaging machine according to the present inventions need not be designed in the above specified way. Instead of being connected to each other, the baffles could be slightly separated. The baffles could also be slightly separated from the side, top, bottom and end walls of the packaging machine. The separation device could also comprise one single baffle with a configuration adapted for achievement of the desired object, or more than two baffles interacting in an appropriate way. Additionally, the baffles need not be orientated in the above specified way. According to alternative embodiments, the baffles could be arranged inclined in relation to the transport direction and a direction orthogonal thereto, respectively.

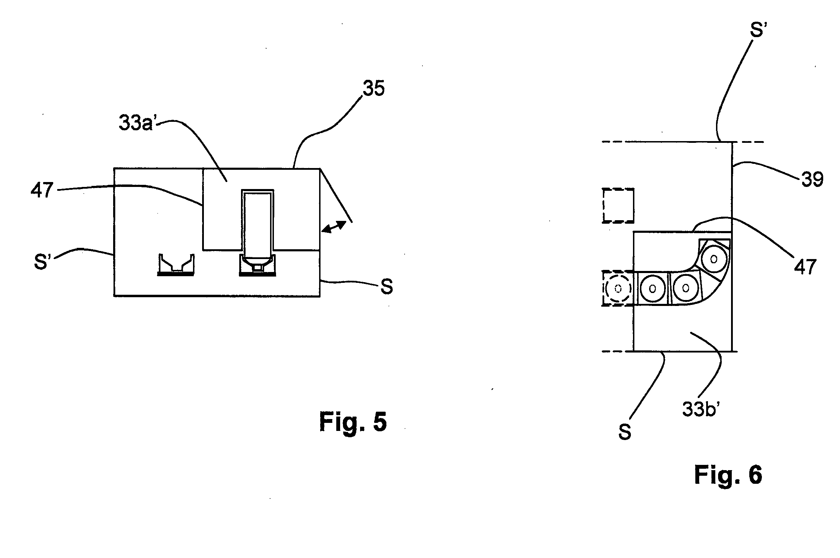

Also, in the above described example, the extension of the final folding zone is defined by the baffles, the plate and the side and top walls of the packaging machine. However, there are numerous other possible solutions. For example, the packaging machine could comprise additional baffles for defining the extension of the final folding zone. As an example, the packaging machine could comprise a third baffle 47 arranged essentially perpendicular to first and second baffles 33a' and 33b' (with alternative designs), respectively, i.e. essentially parallel to the side walls S and S', respectively, of the packaging machine, as is illustrated in figures 5 and 6. This third baffle 47 could extend from the first baffle 33a' to the plate 39 and from the second baffle 33b' to the top wall 35 of the packaging machine.

Further, in the above described example, the buffer zone encloses the final forming zone on two sides, the buffer zone separating the final forming zone from the bottom wall of the packaging machine in the vertical direction and from the sealing zone in the horizontal direction. Naturally, other configurations are possible. As an example, the first baffle 33a could be arranged close to the wall of the duct 27 to so that the buffer zone would not exist between the sealing and final folding zones and the final folding zone would communicate directly with sealing zone.

The above specified pressure condition is just exemplary and alternatives to this are possible. As an example, the pressures inside the final folding zone and the outfeed station could be essentially the same, i.e. P2=P5. It should also be stressed that the pressure within one and the same zone or station need not be constant byt may vary.

Finally, the inventive packaging machine may of course be used for producing other packages than carton bottles.

For the sake of simplicity, the carton bottles, irrespective of which operations that have been performed on them, have been depicted in the same, most simple way throughout the figures.

It should be stressed that components not necessary for describing the present invention has been omitted, both in the figures and in the text, and that the figures are not drawn according to scale.

## Claims

1. A packaging machine (1) comprising a filling zone (9) for filling packages (19) through a respective open (29) end thereof, a sealing zone (11) for sealing said respective open end of the packages after filling, a final folding zone (13) for forming the packages after sealing and a conveyor (17) for transporting the packages through said zones in a transport direction (T), **characterized in that**
the conveyor is arranged to run outside the final folding zone and instead run through a buffer zone (15) to transport the packages through the final folding zone, and
it further comprises means for maintaining a first pressure (P1) inside the sealing zone, a second pressure (P2) inside the final folding zone and a third pressure (P3) inside the buffer zone, the first and third pressures being higher than the second pressure which, in turn, is higher than a fourth pressure (P4) prevailing outside the packaging machine.

2. A packaging machine (1) according to claim 1, further comprising an outfeed station (23) for output of the sealed, filled and formed packages (19), said means being further arranged to maintain a fifth pressure (P5) inside the outfeed station which is higher than the fourth pressure (P4) prevailing outside the packaging machine and lower than the second and third pressures (P2 and P3) inside the final folding zone (13) and buffer zone (15), respectively.

3. A packaging machine (1) according any one of the preceding claims, wherein the final folding zone (13) occupies an upper part thereof and the buffer zone (15) occupies a lower part thereof, said lower part being arranged below said upper part.

4. A packaging machine (1) according to claim 3, wherein the buffer zone (15) also occupies a portion thereof arranged between the sealing zone (11) and the upper and lower parts thereof, the buffer zone delimiting the final folding zone (13) from the sealing zone.

5. A packaging machine (1) according to any one of the preceding claims, wherein said means includes a supply device (25) for supplying a flow of cleaned air to the packaging machine outside the final folding zone (13), and a separation device (33) for physically delimiting the final folding zone from the rest of the zones, said separation device having an opening (41) for enabling passage of the packages (19) through the final folding zone.

6. A packaging machine (1) according to claim 5, wherein the separation device (33) comprises a first baffle (33a, 33a') and a second baffle (33b, 33b') delimiting the final folding zone (13) from the buffer zone (15).

7. A packaging machine (1) according to claim 6, wherein the first baffle (33a, 33a') extends essentially orthogonally to the transport direction (T) and the second baffle (33b, 33b') extends essentially parallel to the transport direction (T).

8. A packaging machine (1) according to any one of claims 6 or 7, wherein the first baffle (33a, 33a') extends from a top wall (35) towards a bottom wall (37) of thereof, whereas the second baffle (33b, 33b') extends from the first baffle in the transport direction (T).

9. A packaging machine (1) according to any one of claims 6-8, wherein the first (33a) and second baffles (33b) extend essentially all the way from one side wall (S) to another side wall (S') of the packaging machine.

10. A packaging machine (1) according to any of the preceding claims, further comprising a hatch (45) in an area of the final folding zone (13) for external access thereto.

11. A packaging method comprising filling, in a filling zone (9), packages (19) through a respective open end (29) thereof, sealing, in a sealing zone (11), said respective open end of the packages after filling, forming, in a final folding zone (13), the packages after sealing and transporting the packages on a conveyor (17) through said zones in a transport direction (T), said zones being comprised in a packaging machine (1), **characterized in that** it further comprises
running the conveyor outside the final folding zone and instead running it through a buffer zone (15) to transport the packages through the final folding zone,and
maintaining a first pressure (P1) inside the sealing zone, a second pressure (P2) inside the final folding zone and a third pressure (P3) inside the buffer zone, the first and third pressures being higher than the second pressure which, in turn, is higher than a fourth pressure (P4) prevailing outside the packaging machine.

12. A packaging method according to claim 11, further comprising outputting, at an outfeed station (23), the sealed, filled and formed packages (19), and maintaining a fifth pressure (P5) inside the outfeed station which is higher than the fourth pressure (P4) prevailing outside the packaging machine and lower than the second and third pressures (P2 and P3) inside the final folding zone (13) and buffer zone (15), respectively.

13. A packaging method according to any one of claims 11 or 12, comprising supplying a flow of cleaned air to the packaging machine (1) outside the final folding zone (13) and providing a separation device (33) for physically delimiting the final folding zone from the rest of the zones, said separation device having an opening (41) for enabling passage of the packages (19) through the final folding zone.

14. A packaging method according to claim 13, wherein the separation device (33) comprises a first baffle (33a) and a second baffle (33b) delimiting the final folding zone (13) from the buffer zone (15).

15. A packaging method according to any one of claims 11-14, further comprising providing a hatch (45) in an area of the final folding zone (13) for external access thereto.

## Patentansprüche

1. Verpackungsmaschine (1), die eine Befüllungszone (9) zum Befüllen von Packstücken (19) durch ein jeweiliges offenes Ende (29) davon, eine Versiegelungszone (11) zum Versiegeln des jeweiligen offenen Endes der Packstücke nach dem Befüllen, eine Endfaltzone (13) zum Formen der Packstücke nach dem Versiegeln sowie eine Fördereinrichtung (17) zum Transportieren der Packstücke durch die Zonen in einer Transportrichtung (T) umfasst, **dadurch gekennzeichnet, dass**
die Fördereinrichtung so angeordnet ist, dass sie sich außerhalb der Endfaltzone befindet und stattdessen durch eine Pufferzone (15) verläuft, um die Packstücke durch die Endfaltzone zu transportieren, und
sie weiterhin Mittel zum Aufrechterhalten eines ersten Drucks (P1) im Innern der Versiegelungszone, eines zweiten Drucks (P2) im Innern der Endfaltzone sowie eines dritten Drucks (P3) im Innern der Pufferzone umfasst, wobei der erste und der dritte Druck höher als der zweite Druck sind, der wiederum höher als ein vierter Druck (P4) ist, der außerhalb der Verpackungsmaschine herrscht.

2. Verpackungsmaschine (1) nach Anspruch 1, die weiterhin eine Ausschleusungsstation (23) zum Ausschleusen der versiegelten, befüllten und geformten Packstücke (19) umfasst, wobei die Mittel weiterhin vorgesehen sind, um einen fünften Druck (P5) im Innern der Ausschleusungsstation aufrechtzuerhalten, der höher als der außerhalb der Verpackungsmaschine herrschende vierte Druck (P4) und niedriger als der zweite und dritte Druck (P2 und P3) im Innern der Endfaltzone (13) bzw. der Pufferzone (15) ist.

3. Verpackungsmaschine (1) nach einem der vorstehend aufgeführten Ansprüche, bei der die Endfaltzone (13) einen oberen Teil davon und die Pufferzone (15) einen unteren Teil davon belegt, wobei der untere Teil unterhalb des oberen Teils angeordnet ist.

4. Verpackungsmaschine (1) nach Anspruch 3, bei der die Pufferzone (15) auch einen Abschnitt davon belegt, der zwischen der Versiegelungszone (11) und dem oberen und unteren Teil davon angeordnet ist, wobei die Pufferzone die Endfaltzone (13) von der Versiegelungszone abgrenzt.

5. Verpackungsmaschine (1) nach einem der vorstehend aufgeführten Ansprüche, bei der die Mittel eine Zuführungsvorrichtung (25), um der Verpackungsmaschine außerhalb der Endfaltzone (13) einen Strom gereinigter Luft zuzuführen, sowie eine Trennvorrichtung (33) beinhalten, um die Endfaltzone von den restlichen Zonen technisch abzugrenzen, wobei die Trennvorrichtung eine Öffnung (41) hat, die ein Passieren der Packstücke (19) durch die Endfaltzone ermöglicht.

6. Verpackungsmaschine (1) nach Anspruch 5, bei der die Trennvorrichtung (33) ein erstes Prallblech (33a, 33a') und ein zweites Prallblech (33b, 33b') umfasst, um die Endfaltzone (13) von der Pufferzone (15) abzugrenzen.

7. Verpackungsmaschine (1) nach Anspruch 6, bei der sich das erste Prallblech (33a, 33a') im Wesentlichen rechtwinklig zur Transportrichtung (T) und das zweite Prallblech (33b, 33b') sich im Wesentlichen parallel zur Transportrichtung (T) erstrecken.

8. Verpackungsmaschine (1) nach einem der Ansprüche 6 oder 7, bei der das erste Prallblech (33a, 33a') sich von einer oberen Wand (35) hin zu einer Bodenwand (37) davon erstreckt, während das zweite Prallblech (33b, 33b') sich vom ersten Prallblech aus in der Transportrichtung (T) erstreckt.

9. Verpackungsmaschine (1) nach einem der Ansprüche 6 bis 8, bei der das erste Prallblech (33a) und das zweite Prallblech (33b) sich im Wesentlichen über die gesamte Erstreckung von einer Seitenwand (S) zu einer anderen Seitenwand (S') der Verpackungsmaschine erstrecken.

10. Verpackungsmaschine (1) nach einem der vorstehend aufgeführten Ansprüche, die weiterhin eine Luke (45) in einem Bereich der Endfaltzone (13) umfasst, um einen externen Zugang dazu zu ermöglichen.

11. Verpackungsverfahren, das das Befüllen, in einer Befüllungszone (9), von Packstücken (19) durch ein jeweiliges offenes Ende (29) davon, das Versiegeln, in einer Versiegelungszone (11), des jeweiligen offenen Endes der Packstücke nach dem Befüllen, das Formen, in einer Endfaltzone (13), der Packstücke nach dem Versiegeln sowie das Transportieren der Packstücke auf einer Fördereinrichtung (17) durch die Zonen in einer Transportrichtung (T) umfasst, wobei die Zonen in einer Verpackungsmaschine (1) zusammengefasst sind, **dadurch gekennzeichnet, dass** es weiterhin Folgendes umfasst:
Verlaufen der Fördereinrichtung außerhalb der Endfaltzone und stattdessen Verlaufen dieser Einrichtung durch eine Pufferzone (15), um die Packstücke durch die Endfaltzone zu transportieren, und
Aufrechterhalten eines ersten Drucks (P1) im Innern der Versiegelungszone, eines zweiten Drucks (P2) im Innern der Endfaltzone sowie eines dritten Drucks (P3) im Innern der Pufferzone, wobei der erste und der dritte Druck höher als der zweite Druck sind, der wiederum höher als ein vierter Druck (P4) ist, der außerhalb der Verpackungsmaschine herrscht.

12. Verpackungsverfahren nach Anspruch 11, das weiterhin, an einer Ausschleusungsstation (23), das Ausschleusen der versiegelten, befüllten und geformten Packstücke (19) sowie das Aufrechterhalten eines fünften Drucks (P5) im Innern der Ausschleusungsstation umfasst, der höher als der außerhalb der Verpackungsmaschine herrschende vierte Druck (P4) und niedriger als der zweite und dritte Druck (P2 und P3) im Innern der Endfaltzone (13) bzw. der Pufferzone (15) ist.

13. Verpackungsverfahren nach einem der Ansprüche 11 oder 12, das das Zuführen eines Stroms gereinigter Luft zur Verpackungsmaschine (1) außerhalb der Endfaltzone (13) sowie das Bereitstellen einer Trennvorrichtung (33) zur technischen Abgrenzung der Endfaltzone von den restlichen Zonen umfasst, wobei die Trennvorrichtung eine Öffnung (41) hat, die das Passieren der Packstücke (19) durch die Endfaltzone ermöglicht.

14. Verpackungsverfahren nach Anspruch 13, bei dem die Trennvorrichtung (33) ein erstes Prallblech (33a) und ein zweites Prallblech (33b) zur Abgrenzung der Endfaltzone (13) von der Pufferzone (15) umfasst.

15. Verpackungsverfahren nach einem der Ansprüche 11 - 14, das weiterhin das Bereitstellen einer Luke (45) in einem Bereich der Endfaltzone (13) umfasst, um einen externen Zugang dazu zu ermöglichen.

## Revendications

1. Machine d'emballage (1) comprenant une zone de remplissage (9) pour remplir des emballages (19) à travers une extrémité ouverte respective (29) de ceux-ci, une zone de scellement (11) pour sceller ladite extrémité ouverte respective des emballages après leur remplissage, une zone de pliage final (13) pour former les emballages après leur scellement et un transporteur (17) pour transporter les emballages à travers lesdites zones dans une direction de transport (T), **caractérisée en ce que**
le transporteur est prévu pour passer à l'extérieur de la zone de pliage final et pour passer à la place par une zone tampon (15) pour transporter les emballages à travers la zone de pliage final, et
elle comprend en outre un moyen pour maintenir une première pression (P1) à l'intérieur de la zone de scellement, une deuxième pression (P2) à l'intérieur de la zone de pliage final et une troisième pression (P3) à l'intérieur de la zone tampon, les première et troisième pressions étant supérieures à la deuxième pression, qui à son tour, est supérieure à une quatrième pression (P4) régnant à l'extérieur de la machine d'emballage.

2. Machine d'emballage (1) selon la revendication 1, comprenant en outre un poste de décharge (23) pour décharger les emballages (19) scellés, remplis et formés, ledit moyen étant en outre prévu pour maintenir une cinquième pression (P5) à l'intérieur du poste de décharge, laquelle est supérieure à la quatrième pression (P4) régnant à l'extérieur de la machine d'emballage et inférieure aux deuxième et troisième pressions (P2 et P3) à l'intérieur de la zone de pliage final (13) et de la zone tampon (15), respectivement.

3. Machine d'emballage (1) selon l'une quelconque des revendications précédentes, dans laquelle la zone de pliage final (13) occupe une partie supérieure de celle-ci et la zone tampon (15) occupe une partie inférieure de celle-ci, ladite partie inférieure étant disposée en dessous de ladite partie supérieure.

4. Machine d'emballage (1) selon la revendication 3, dans laquelle la zone tampon (15) occupe également une partie de celle-ci disposée entre la zone de scellement (11) et les parties supérieure et inférieure de celle-ci, la zone tampon délimitant la zone de pliage final (13) par rapport à la zone de scellement.

5. Machine d'emballage (1) selon l'une quelconque des revendications précédentes, dans laquelle ledit moyen comporte un dispositif d'alimentation (25) pour acheminer un flux d'air propre à la machine d'emballage à l'extérieur de la zone de pliage final (13), et un dispositif de séparation (33) pour délimiter physiquement la zone de pliage final par rapport au reste des zones, ledit dispositif de séparation ayant une ouverture (41) permettant le passage des emballages (19) à travers la zone de pliage final.

6. Machine d'emballage (1) selon la revendication 5, dans laquelle le dispositif de séparation (33) comprend une première chicane (33a, 33a') et une deuxième chicane (33b, 33b') délimitant la zone de pliage final (13) par rapport à la zone tampon (15).

7. Machine d'emballage (1) selon la revendication 6, dans laquelle la première chicane (33a, 33a') s'étend essentiellement perpendiculairement à la direction de transport (T) et la deuxième chicane (33b, 33b') s'étend essentiellement parallèlement à la direction de transport (T).

8. Machine d'emballage (1) selon l'une quelconque des revendications 6 ou 7, dans laquelle la première chicane (33a, 33a') s'étend depuis une paroi supérieure (35) vers une paroi inférieure (37) de celle-ci, tandis que la deuxième chicane (33b, 33b') s'étend depuis la première chicane dans la direction de transport (6).

9. Machine d'emballage (1) selon l'une quelconque des revendications 6 à 8, dans laquelle les première (33a) et deuxième (33b) chicanes s'étendent essentiellement sur toute la distance depuis une paroi latérale (S) jusqu'à une autre paroi latérale (S') de la machine d'emballage.

10. Machine d'emballage (1) selon l'une quelconque des revendications précédentes, comprenant en outre une trappe (45) dans une partie de la zone de pliage final (13) permettant un accès à celle-ci depuis l'extérieur.

11. Procédé d'emballage comprenant le remplissage, dans une zone de remplissage (9), d'emballages (19) à travers une extrémité ouverte respective (29) de celle-ci, le scellement, dans une zone de scellement (11), de ladite extrémité ouverte respective des emballages après leur remplissage, le formage, dans une zone de pliage final (13), des emballages après le scellement et le transport des emballages sur un transporteur (17) à travers lesdites zones dans une direction de transport (T), lesdites zones étant comprises dans une machine d'emballage (1), **caractérisé en ce qu'**il comprend en outre
le passage du transporteur à l'extérieur de la zone de pliage final et son passage à la place à travers une zone tampon (15) pour transporter les emballages à travers la zone de pliage final, et le maintien d'une première pression (P1) à l'intérieur de la zone de scellement, d'une deuxième pression (P2) à l'intérieur de la zone de pliage final et d'une troisième pression (P3) à l'intérieur de la zone tampon, les première et troisième pressions étant supérieures à la deuxième pression qui, à son tour, est supérieure à une quatrième pression (P4) régnant à l'extérieur de la machine d'emballage.

12. Procédé d'emballage selon la revendication 11, comprenant en outre le déchargement, au niveau d'un poste de décharge (23), des emballages scellés, remplis et formés (19), et le maintien d'une cinquième pression (P5) à l'intérieur du poste de décharge, laquelle est supérieure à la quatrième pression (P4) régnant à l'extérieur de la machine d'emballage et inférieure aux deuxième et troisième pressions (P2 et P3) à l'intérieur de la zone de pliage final (13) et de la zone tampon (15), respectivement.

13. Procédé d'emballage selon l'une quelconque des revendications 11 ou 12, comprenant l'acheminement d'un flux d'air propre à la machine d'emballage (1) à l'extérieur de la zone de pliage final (13) et la fourniture d'un dispositif de séparation (33) pour délimiter physiquement la zone de pliage final par rapport au reste des zones, ledit dispositif de séparation ayant une ouverture (41) pour permettre le passage des emballages (19) à travers la zone de pliage final.

14. Procédé d'emballage selon la revendication 13, dans lequel le dispositif de séparation (33) comprend une première chicane (33a) et une deuxième chicane (33b) délimitant la zone de pliage final (13) par rapport à la zone tampon (15).

15. Procédé d'emballage selon l'une quelconque des revendications 11 à 14, comprenant en outre la fourniture d'une trappe (45) dans une partie de la zone de pliage final (13) pour permettre un accès à celle-ci depuis l'extérieur.
